# EUROPEAN PATENT APPLICATION

(11) **EP 3 184 516 A1**
(43) Date of publication of application: **28.06.2017**
(21) Application number: 15382661.5
(22) Date of filing: 23.12.2015
(51) Int. Cl.: C07D 277/40, A61K 31/426, A61P 3/10, A61P 13/10

(54) **CRYSTALLINE INCLUSION COMPLEXES OF MIRABEGRON WITH BETA-CYCLODEXTRIN**

(71) Applicant: Enantia, S.L., 08028 Barcelona (ES)
(72) Inventor: TESSON, Nicolas, 08028 BARCELONA (ES); JIMÉNEZ GONZÁLEZ, Carme, 08028 BARCELONA (ES)
(74) Representative: ZBM Patents - Zea, Barlocci & Markvardsen

(57) **Abstract**

Crystalline inclusion complexes of 2-(2-Amino-1,3-thiazol-4-yl)-N-[4-(2-{[(2R)-2-hydroxy-2-phenylethyl]amino}ethyl)phenyl]acetamide and β-cyclodextrin, in particular crystalline Forms I, II, or mixtures thereof, their preparation processes, pharmaceutical compositions comprising them, as well as their therapeutical indication.

## Description

The present invention relates to crystalline inclusion complexes of Mirabegron, in particular, to crystalline inclusion complexes of Mirabegron with β-cyclodextrin, processes for their preparation, and their use as medicaments. It also relates to pharmaceutical compositions comprising them.

### BACKGROUND ART

Mirabegron is the International Nonproprietary Name (INN) of 2-(2-amino-1,3-thiazol-4-yl)-N-[4-(2-{[(2R)-2-hydroxy-2-phenylethyl]amino}ethyl)phenyl]acetamide. Mirabegron activates the β3 adrenergic receptor in the detrusor muscle in the bladder, which leads to muscle relaxation and an increase in bladder capacity. It is a drug developed by Astellas Pharma for the treatment of overactive bladder. The structure of Mirabegron corresponds to formula (I) below.

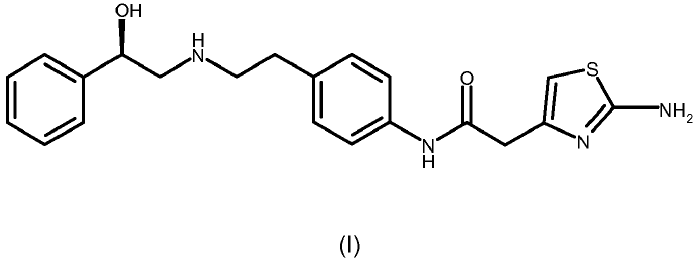

Mirabegron was first described in the patent family of the EP1028111A1. In particular, it was disclosed in Example 41 of the previous document as a dihydrochloride salt.

According to EP1440969A1, from the same applicant, the dihydrochloride salt is a strongly hygroscopic and unstable crystal. A drawback of hygroscopic compounds is that the water content is different depending upon lots which make them unsuitable for formulation uses.

Two different crystal forms of Mirabegron free base, named as α-form and β-form, were also disclosed in EP1440969A1. According to this document, the α-form is stable and non-hygroscopic. Unfortunately, this form is practically insoluble in water. Mirabegron free base is disclosed as being practically insoluble in water (0.082 mg/mL) (cf. *Betmiga Mirabegron report* (AusPar), *Myrbetriq Highlights of prescribing information* (FDA). Solubility tests performed by the present inventors indicated a solubility in water at room temperature of less than 50 volumes. Mirabegron also degrades under hydrolytic and oxidative stress conditions while it is stable under thermal and photolytic conditions (cf. "Characterization of stress degradation products of mirabegron using UPLC-QTOF-MS/MS and in silico toxicity predictions of its degradation products", P. D. Kalariya et al., RSC Adv., 2015,5, 31024-31038). On the other hand, the β-form is a metastable form with low hygroscopicity. The reported use of this last Form is as an intermediate for the preparation of the α-Form.

WO2012156998A2 discloses amorphous Mirabegron free base as well as a solid dispersion of amorphous Mirabegron together with one or more pharmaceutically acceptable carriers.

Different solid forms can have different characteristics, and offer certain advantages, for example with regard to solubility and/or bioavailability. It is well known that solubility of an active ingredient may influence its bioavailability. In addition, different solid forms also may show differences in other pharmaceutical properties such as stability, hygroscopicity, flowability, or compressibility. Some forms are more adequate for one type of formulation, and other forms for other different formulations. Furthermore, depending on the therapeutic indications, one or another pharmaceutical formulation may be preferred.

On the other hand, it is known that water solubility of drugs may be increased by forming complexes or adducts of compounds with cyclodextrins, generally, significantly enhanced solubility is obtained only with high molar ratios cyclodextrin/active substance. In many cases, solutions of over 10% (wt/vol) of cyclodextrin are used to obtain a significant solubility of the drug (cf. "Pharmaceutical Applications of Cyclodextrins. 1. Drug Solubilization and Stabilization", T. Loftsson et al. J. Pharm. Sc. 1996, vol. 85, pp.1017-1025). Said drug/cyclodextrin ratios are suitable for parenteral administration or aqueous eye drops but the high relative amount of cyclodextrin respect to drug make these technically unsuitable for the preparation of tablet or capsules for oral administration. Numerous inclusion complexes of pharmaceutically active ingredients with cyclodextrins described in the literature indicate amorphous or low crystalline solid phases more prone to possible chemical degradations than crystalline phases. Furthermore methods of preparations of these complexes such as spray draying, kneading, freeze drying or lyophylization favor the formations of very small particles (such as nanoparticles) or amorphous phases leading to a overestimation of solubility improvement caused by cyclodextrin complexation effect.

From what is known in the art, there is still the need of finding new solid forms of Mirabegron with high solubility in water and high stability, for improving the pharmaceutical properties of the pharmaceutical formulations containing them.

### SUMMARY OF THE INVENTION

Inventors have found high crystalline inclusion complexes of Mirabegron and β-cyclodextrin. In particular, these crystalline forms correspond to a family of hydrates having different water ratio. The provision of crystalline inclusion complexes of Mirabegron with β-cyclodextrin give a new tool to overcome the problems associated with suboptimal properties of the known forms of Mirabegron free base and of Mirabegron hydrochloride. The stable crystalline inclusion complexes of Mirabegron of the present invention have a surprisingly high solubility in water at room temperature, much higher than it would have been expected for an inclusion complex. The high solubility in water of the crystalline inclusion complexes of Mirabegron of the present invention provide enhanced bioavailability and enhanced galenic properties to the pharmaceutical formulations containing them.

The formation of an inclusion complex implies an increase in the weight of the active ingredient. An increase in formulation volume represents a critical stage in the applicability of cycoldextrin inclusion complexes for oral solid dosage forms. Advantageously, the specific crystalline inclusion complexes of Mirabegron with β-cyclodextrin of the present invention has a reduced amount of β-cyclodextrin (molar ratio 1:1). On the other hand, Mirabegron is generally administered in low drug doses, in particular tablets containing 25 or 50 mg of Mirabegron. Therefore, the crystalline inclusion complexes of Mirabegron and β-cyclodextrin of the present invention are highly appropriate to the formulation of solid dosage forms containing them.

The new stable solid forms with increased solubility may also be useful for the preparation of other crystalline forms, since the high solubility in water make them useful for purification purposes, so they can be isolated using crystallization processes in order to obtain the desired purity specifications, in particular, Mirabegron with high chemical purity.

Accordingly, an aspect of the present invention relates to the provision of crystalline forms of an inclusion complex of Mirabegron with β-cyclodextrin.

Another aspect of the present invention relates to crystalline inclusion complexes of Mirabegron and β-cyclodextrin for use as a medicament.

Another aspect of the present invention relates to crystalline inclusion complexes of Mirabegron and β-cyclodextrin for use in the prevention and/or treatment of overactive bladder.

Another aspect of the present invention relates to a pharmaceutical composition comprising a therapeutically effective amount of a crystalline inclusion complex of Mirabegron and β-cyclodextrin, in particular Form I or Form II or mixtures thereof, together with appropriate amounts of pharmaceutical excipients or carriers.

Finally, these crystalline forms are prepared in an aqueous medium. Different drying conditions allow selecting the desired hydrated form. Thus, another aspect of the present invention relates to several processes to prepare the crystalline inclusion complexes of Mirabegron and β-cyclodextrin of the present invention.

### DETAILED DESCRIPTION OF THE INVENTION

For the sake of understanding, the following definitions are included and expected to be applied throughout description, claims and drawings.

An inclusion compound is a complex in which one chemical compound (the "host") forms a cavity in which molecules of a second "guest" compound are located. The host and guest molecules are joined by weak interactions. The Inclusion complexes of the present invention are formed between β-cyclodextrin and Mirabegron as guest molecule.

The term "weak interaction" refers herein as an interaction which is neither ionic nor covalent, and includes for example: hydrogen bonds, van der Waals interactions, and π-π stacking.

The term "solvate" is to be understood as meaning any form of the crystalline form in which the compound has attached to it via non-covalent binding solvent molecules. When the solvent is water the solvate is a hydrate.

When a ratio of components of the crystalline forms of the inclusion complexes of Mirabegron of the present invention is specified it refers to the molar ratio between the Mirabegron and the further component (β-cyclodextrin) that forms the inclusion complex. Thus, the term "molar ratio" has been used to express the stoichiometric amount in moles of each of the components of the inclusion complex.

For the purposes of the invention, any ranges given include both the lower and the upper end-points of the range. Ranges given, such as temperatures, times, and the like, should be considered approximate, unless specifically stated.

When values of characteristic peaks of an X-ray diffractogram are given it is said that are "approximate" values. It should be understood that the values are the ones shown in the corresponding lists or tables ± 0.1 degrees 2 theta measured in an X-ray diffractometer with Cu-K_{α} radiation λ=1.5406 Å.

The term "room temperature" as disclosed herein refers to a temperature of the environment, without heating or cooling, and is generally comprised of from 20 to 25 °C.

For the term "pure crystalline form" is understood that the crystallline form has a percentage by weight equal to or lower than 5% of any other crystalline form, preferably, equal to or lower than 2% by weight, more preferably, equal to or lower than 1% by weight.

The expression "crystalline form obtainable by" is used here to define each specific crystalline form of the invention by the process for obtaining it and refers to the product obtainable by any of the corresponding processes disclosed herein. For the purposes of the invention the expressions "obtainable", "obtained" and equivalent expressions are used interchangeably, and in any case, the expression "obtainable" encompasses the expression "obtained".

The term "therapeutically effective amount" as used herein, refers to the amount of a compound that, when administered, is sufficient to prevent development of, or alleviate to some extent, one or more of the symptoms of the illness to be treated. The particular dose of compound administered according to this invention will of course be determined by the particular circumstances surrounding the case, including the compound administered, the route of administration, the particular condition being treated, and the similar considerations.

The term "pharmaceutical composition" refers to a mixture of a compound disclosed herein with other chemical components, such as diluents or carriers. The pharmaceutical composition facilitates administration of the compound to an organism.

The term "pharmaceutically acceptable excipients or carriers" refers to pharmaceutically acceptable material, composition or vehicle, such as liquid or solid filler, diluent, excipient, solvent, or encapsulating material. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The term "treatment" meant to include alleviating or eradicating a disorder, disease, or condition, or one or more of the symptoms associated with the disorder, disease or condition, or alleviating or eradicating the cause(s) of the disorder, disease, or condition itself.

As mentioned above, an aspect of the present invention relates to the provision of crystalline inclusion complexes of Mirabegron with β-cyclodextrin.

Cyclodextrins are hydrophilic cyclic oligosaccharides with a lipophilic central cavity. The β-cyclodextrin is a 7-membered sugar ring molecule of formula:

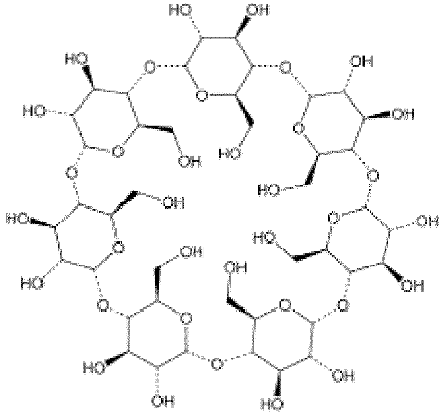

The safety profile of natural cyclodextrins and their derivatives has been widely studied, and they have generally proven to be atoxic, because they only manage to cross biological membranes with some degree of difficulty. The regulatory status of cyclodextrins is evolving. β-cyclodextrin is listed in a number of pharmacopoeia sources including the US Pharmacopoeia.

In a particular embodiment, the crystalline inclusion complexes of Mirabegron and β-cyclodextrin of the present invention are hydrate forms.

In a preferred embodiment, a crystalline inclusion complex of Mirabegron with β-cyclodextrin of the present invention is named Form I. This form is a stable form in the stability conditions tested (Room temperature, RT - 40-60 % relative humidity, RH) and also in suspension in pure water at room temperature.

The crystalline inclusion complex of Mirabegron Form I has been characterized by X-Ray Pattern Diffractogram, XRPD, (FIG. 1); Proton Nuclear Magnetic Resonance analyses, ¹H NMR, (FIG. 3); Differential Scanning Calorimetry, DSC, (FIG. 5); and Karl Fischer (KF).

The Mirabegron : β-cyclodextrin ratio of crystalline inclusion complex of Mirabegron Form I can be determined by ¹H NMR. The molar ratio of this crystalline form is 1:1.

The X-ray diffraction confirms the formation of the complex since the obtained peaks were not the result of the sum of the peaks related to the two separate components.

In a preferred embodiment, the crystalline inclusion complex of Mirabegron and β-cyclodextrin Form I is characterized by having an X-ray diffractogram that comprises characteristic peaks at approximately 7.2, 9.9, 12.0, 14.5, 15.7, 17.6, 18.5, 18.8, 20.7, and 23.8 degrees 2 theta at a Cu-K_{α} radiation, A = 1.5406 Å.

In a more preferred embodiment, the crystalline inclusion complex of Mirabegron and β-cyclodextrin Form I is characterized by having an X-ray diffractogram that further comprises characteristic peaks at approximately 6.0, 7.2, 9.9, 12.0, 14.5, 15.7, 17.6, 17.7, 18.5, 18.8, 19.7, 20.7, 21.4, 22.6, 23.3, 23.8, 25.9, 29.4, 36.1, and 38.1 degrees 2 theta at a Cu-K_{α} radiation, λ = 1.5406 Å.

More specifically, the crystalline inclusion complex of Mirabegron and β-cyclodextrin Form I is characterized by exhibiting in the powder X-ray diffractogram a pattern of peaks, expressed in 2 theta units in degrees, 2θ (°), which is shown in Table 1.

**Table 1: List of selected peaks of XRPD (only peaks with relative intensity greater than or equal to 1% are indicated):**

| Pos. [°2Th.] | Rel. Int. [%] |
|---|---|
| 3,2 | 1 |
| 6,0 | 37 |
| 7,2 | 19 |
| 8,0 | 1 |
| 9,6 | 12 |
| 9,9 | 24 |
| 11,8 | 41 |
| 12,0 | 100 |
| 12,6 | 6 |
| 14,0 | 3 |
| 14,5 | 17 |
| 15,0 | 9 |
| 15,3 | 17 |
| 15,7 | 15 |
| 16,0 | 3 |
| 17,6 | 18 |
| 17,7 | 9 |
| 18,1 | 5 |
| 18,3 | 7 |
| 18,5 | 15 |
| 18,8 | 18 |
| 19,7 | 7 |
| 20,1 | 2 |
| 20,7 | 9 |
| 21,0 | 2 |
| 21,4 | 7 |
| 22,6 | 4 |
| 23,3 | 4 |
| 23,8 | 11 |
| 24,8 | 2 |
| 25,3 | 2 |
| 25,9 | 5 |
| 27,0 | 2 |
| 28,7 | 1 |
| 29,4 | 3 |
| 29,9 | 1 |
| 33,8 | 1 |
| 34,3 | 3 |
| 36,1 | 6 |
| 38,1 | 4 |

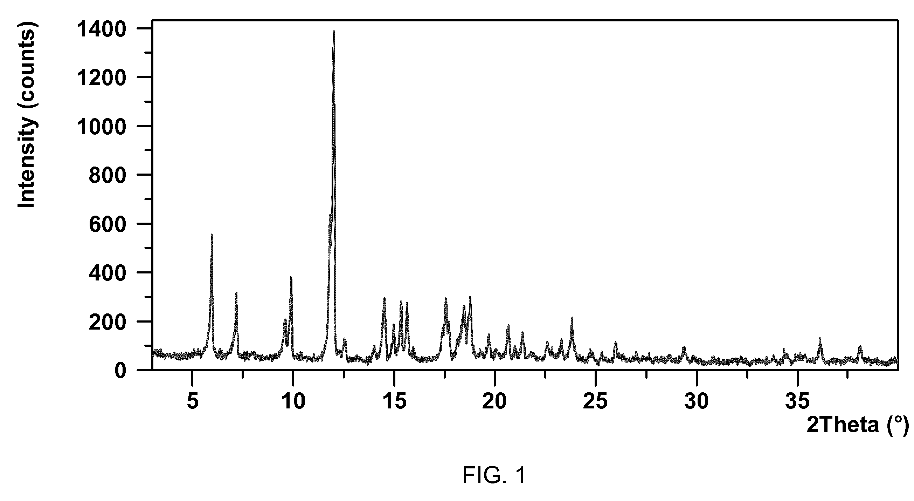

This crystalline inclusion complex of Mirabegron with β-cyclodextrin Form I may be further characterized by an X-ray diffractogram as in FIG. 1.

In a particular embodiment, crystalline inclusion complex of Mirabegron with β-cyclodextrin Form I is also characterized by DSC. DSC curves indicate numerous dehydration thermic events, showing the loss of water starting at room temperature. The melting characteristics of the guest (Mirabegron) are absent in the beta-cyclodextrin inclusion complex Form I.

The TG analysis of Form I shows a progressive weight loss of 7.2% between 25 and 112 °C and 3.7% between 112 and 174 °C (10.9% weight loss corresponding to ca. 10 water molecules). This weight loss matches with the thermal events observed by DSC and with the water content detected by Karl Fisher (10.8%).

K-F analysis of the crystalline inclusion complex of Mirabegron with β-cyclodextrin Form I shows an average 10.5%-11.5% water content corresponding to approximately 10 water molecules.

The crystalline inclusion complex of Mirabegron with β-cyclodextrin Form I is the hydrated form with the higher amount of water at room temperature since a suspension of this crystalline form in water is maintained stable.

The crystalline inclusion complex of Mirabegron with β-cyclodextrin Form I can be obtained by a process comprising crystallizing an inclusion complex of Mirabegron with β-cyclodextrin either from a solution comprising Mirabegron, β-cyclodextrin and water, or from a solution comprising Mirabegron, β-cyclodextrin, and a solvent system which is a mixture of a water miscible solvent/water; followed by filtrating and drying the crystalline inclusion complex of Mirabegron with β-cyclodextrin Form I thus obtained at room temperature under vacuum for the necessary period of time to obtain dry Form I. The necessary period of time may be controlled by K-F. XRPD allows checking any over drying leading to Form II contamination In this case further exposition to humidity allows recovering pure Form I.

The initial solution of Mirabegron can be the prepared from a solution of Mirabegron in an organic solvent, to which water and cyclodextrin is added, followed by azeotropic distillation to remove completely or partially the organic solvent.

The amount of β-cyclodextrin needed to prepare the crystalline inclusion complex of Mirabegron and β-cyclodextrin Form I may range from 1 to 10 equivalents of β-cyclodextrin per equivalent of Mirabegron starting material, preferably from 1.3 to 5 equivalents of β-cyclodextrin per equivalent of Mirabegron starting material. Generally, the amount of β-cyclodextrin used to prepare the crystalline Form I is of from 1.3 to 2 equivalents with respect to Mirabegron starting material. In a preferred embodiment, the amount of β-cyclodextrin used to prepare the crystalline form is of 1.3-1.5 equivalents with respect to 1 equivalent of Mirabegron.

In a preferred embodiment, the solvent used is water. In a particular embodiment of the process, the amount of water is comprised between 10 and 20 ml of water per gram of Mirabegron. In another particular embodiment, the amount of water is 15 ml of water per gram of Mirabegron.

Examples of suitable water miscible solvents that can be used for the preparation of Form I include methanol, ethanol, 1-propanol, 2-propanol, 2-methylpropanol, 1-butanol, 2-butanol, 3-mehtyl-1-butanol, 1-pentanol, 2-pentanol, ethyl formate, dimethylsulfoxide, dioxane, acetonitrile, tetrahydrofuran, N,N-dimethylformamide and N-methylpyrrolidone

Generally, the drying step is carried out at room temperature and under a vacuum of 5-10 mbar until obtained a handled solid with a KF of ca. 10-12%. After the drying step, XRPD was performed in order to confirm the presence of pure Form I. If traces of Form II are detected, an exposition at 40 °C at 75% RH allows to obtain pure Form I (conversion controlled by XRPD).

The process for the preparation of the crystalline inclusion complex of Mirabegron and β-cyclodextrin Form I is advantageous because it can be easily used at industrial scale and yield the inclusion complex of Mirabegron with β-cyclodextrin with good crystallinity. In addition, the crystalline form of Mirabegron and β-cyclodextrin Form I can be obtained using only water (ecofriendly solvent) as solvent.

The crystalline inclusion complex of Mirabegron with β-cyclodextrin Form I can also be obtained by a process comprising slurring Mirabegron in any crystalline form or the amorphous form, β-cyclodextrin, and water at a temperature of from 0°C to 60°C, for the necessary period of time until complete formation to an inclusion complex; followed by filtrating and drying the crystalline inclusion complex of Mirabegron with β-cyclodextrin Form I thus obtained at room temperature under vacuum, in the same particular and preferred conditions disclosed above for the obtention of Form I by crystallization. Preferably the slurring temperature is of from room temperature to 40 °C. More preferably, the slurring temperature is room temperature.

Crystalline inclusion complex of Mirabegron with β-cyclodextrin Form I may also be defined by its preparation process. Accordingly, it is considered part of the invention the crystalline inclusion complex of Mirabegron and β-cyclodextrin Form I obtainable by any of the processes and specific conditions disclosed above.

In another particular embodiment, the crystalline inclusion complex of Mirabegron with β-cyclodextrin is named Form II. This form is also stable form in the stability conditions tested (RT - 40-60 % RH).

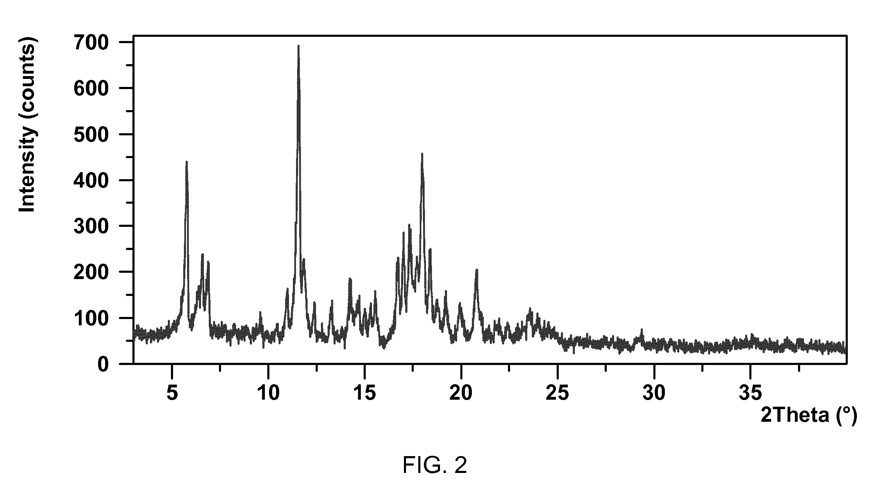

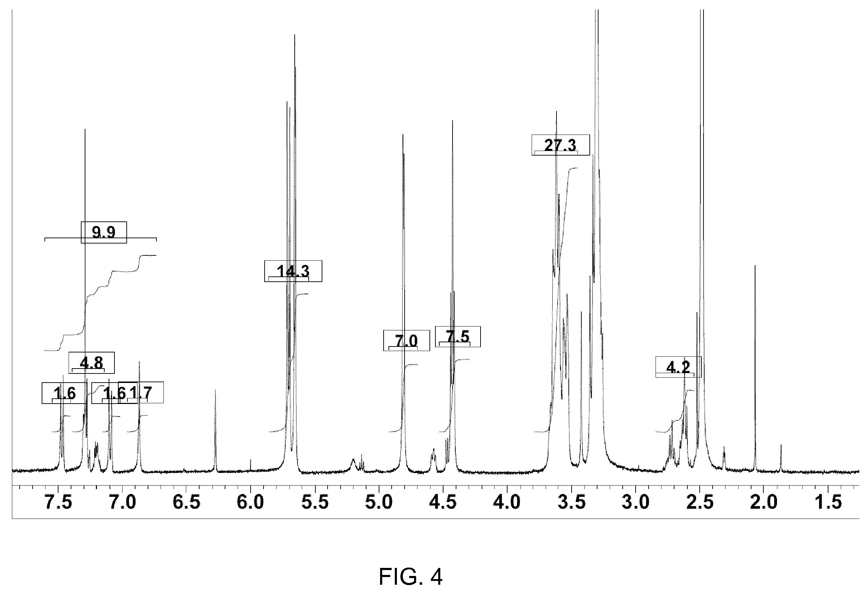

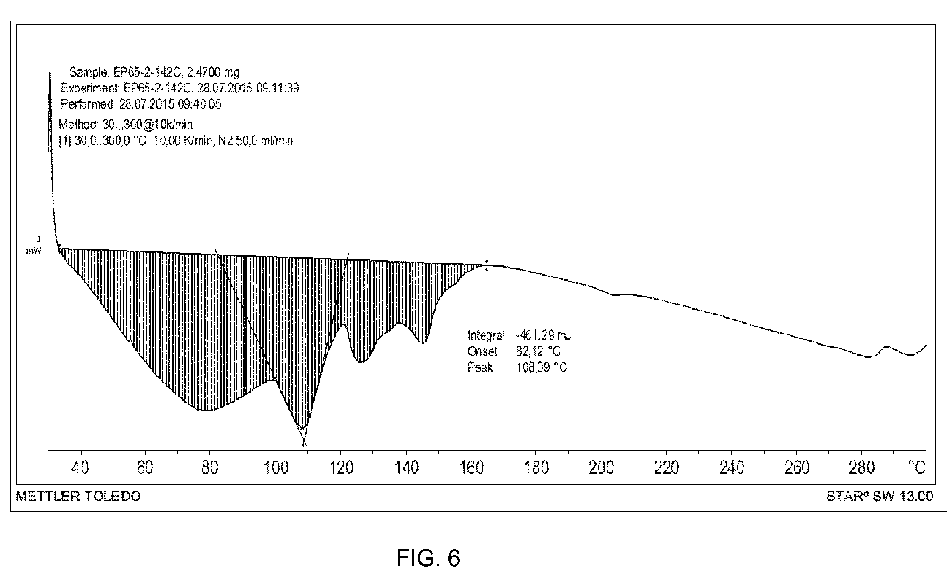

The crystalline inclusion complex of Mirabegron with β-cyclodextrin Form II has been characterized by XRPD (FIG. 2), ¹H NMR (FIG. 4), DSC (see FIG. 6) and Karl Fischer (KF).

The Mirabegron : β-cyclodextrin ratio of the crystalline form of Mirabegron and β-cyclodextrin Form II is determined by ¹H NMR. The molar ratio of this crystalline form is 1:1.

The X-ray diffraction confirms the formation of the complex also for Form II since the obtained peaks were not the result of the sum of the peaks related to the two separate components.

In a preferred embodiment, the crystalline inclusion complex of Mirabegron with β-cyclodextrin Form II is characterized by having an X-ray diffractogram that comprises characteristic peaks at approximately 5.8, 6.6, 6.9, 11.0, 11.6, 11.9, 14.2, 15.5, 16.7, 17.0, 17.4, 18.0, 18.4, 19.2, and 20.8 degrees 2 theta at a Cu-K_{α} radiation, λ = 1.5406 Å.

In a more preferred embodiment, the crystalline inclusion complex of Mirabegron with β-cyclodextrin II Form II is characterized by having an X-ray diffractogram that further comprises characteristic peaks at approximately 5.8, 6.6, 6.9, 11.0, 11.6, 11.9, 13.3, 14.2, 15.0, 15.3, 15.5, 16.7, 17.0, 17.4, 18.0, 18.4, 19.2, 20.8, and 23.5 degrees 2 theta at a Cu-K_{α} radiation, λ = 1.5406 Å.

More specifically, this crystalline inclusion complex of Mirabegron with β-cyclodextrin Form II is characterized by exhibiting in the powder X-ray diffractogram a pattern of peaks, expressed in 2 theta units in degrees, 2θ (°), which is shown in Table 2.

**Table 2: List of selected peaks of XRPD (only peaks with relative intensity greater than or equal to 1% are indicated):**

| Pos. [°2Th.] | Rel. Int. [%] |
|---|---|
| 5,8 | 62 |
| 6,4 | 15 |
| 6,6 | 31 |
| 6,9 | 26 |
| 8,3 | 4 |
| 8,9 | 3 |
| 9,6 | 7 |
| 10,4 | 4 |
| 11,0 | 20 |
| 11,6 | 100 |
| 11,9 | 30 |
| 12,4 | 13 |
| 13,3 | 11 |
| 14,2 | 24 |
| 14,7 | 16 |
| 15,0 | 11 |
| 15,3 | 13 |
| 15,5 | 19 |
| 16,7 | 33 |
| 17,0 | 39 |
| 17,4 | 45 |
| 17,7 | 31 |
| 18,0 | 75 |
| 18,4 | 33 |
| 18,8 | 13 |
| 19,2 | 15 |
| 19,9 | 13 |
| 20,8 | 27 |
| 22,0 | 5 |
| 22,4 | 5 |
| 23,5 | 9 |
| 24,0 | 7 |
| 24,7 | 3 |
| 29,4 | 4 |
| 35,0 | 2 |

This crystalline inclusion complex of Mirabegron with β-cyclodextrin Form II may be further characterized by an X-ray diffractogram as in FIG. 2.

In a particular embodiment, the crystalline inclusion complex of Mirabegron with β-cyclodextrin Form II is also characterized by a DSC which indicates numerous dehydration thermic events. The melting characteristics of the guest (Mirabegron) are absent in the beta-cyclodextrin inclusion complex Forms II.

The TG analysis of Form II shows a progressive weight loss of 4.3% between 25 and 108 °C and 4.1% between 108 and 190 °C (8.3% weight loss corresponding to ca. 8 water molecules). This weight loss matches with the thermal events observed by DSC and with the water content detected by Karl Fisher (8.9%).

K-F analysis of the crystalline inclusion complex of Mirabegron with β-cyclodextrin Form II showed lower water content than for Form I. In particular Form II has an average 8.9% water content, corresponding to ca. 8 water molecules.

The crystalline inclusion complex of Mirabegron with β-cyclodextrin Form II can be obtained by using specific drying conditions starting from the crystalline inclusion complex of Mirabegron with β-cyclodextrin Form I. In particular, this drying step is carried out under vacuum at a temperature of from room temperature to 80 °C until complete transformation, optionally in the presence of a desiccant agent. Preferably the vacuum is of 5-10 mbar. The necessary period of time for the complete transformation may be controlled by K-F or by XRPD.

In a particular embodiment, the desiccant agent is P₂O₅. In a particular embodiment, the desiccant agent provides an environment with a relative humidity equal to or below than 20%.

The crystalline inclusion complex of Mirabegron with β-cyclodextrin Form II can be prepared following the same crystallization step or the same slurring step disclosed for the preparation of the crystalline inclusion complex of Mirabegron with β-cyclodextrin Form I, but using different drying conditions to carry out the drying step of the filtered product. These different drying conditions yield to the obtention of the crystalline inclusion complex of Mirabegron with β-cyclodextrin Form II.

Thus, this form can be obtained by a process comprising a) crystallizing an inclusion complex of Mirabegron with β-cyclodextrin from either a solution comprising Mirabegron, β-cyclodextrin and water, or from a solution comprising Mirabegron, β-cyclodextrin and a solvent system which is a mixture of a water miscible solvent/water, or alternatively, by slurring Mirabegron, β-cyclodextrin, and water at a temperature of from 0°C to 60 °C for the necessary period of time until complete formation of an inclusion complex; and b) followed by filtrating and drying the crystalline inclusion complex of Mirabegron with β-cyclodextrin Form I thus obtained under vacuum at a temperature of from room temperature to 80 °C, for the necessary period of time to observe the complete transformation of Form I to Form II. Preferably, the drying temperature is of from 40°C to 80 °C. The necessary period of time may be controlled by K-F or by XRPD.

The particular and preferred embodiments for carrying out the crystallization step of an inclusion complex of Mirabegron with β-cyclodextrin or for carrying out the slurring step in the process for preparing the crystalline inclusion complex Mirabegron with β-cyclodextrin Form I disclosed above, are also particular and preferred embodiments for the preparation of the crystalline inclusion complex of Mirabegron with β-cyclodextrin Form II.

The process for the preparation of the crystalline inclusion complex of Mirabegron and β-cyclodextrin Form II is advantageous for the same reasons disclosed above for the process for preparing Form I, in particular, it can be easily used at industrial scale, it yields the inclusion complex of Mirabegron with β-cyclodextrin with good crystallinity, and the crystalline form of Mirabegron and β-cyclodextrin Form II can be obtained using only water (ecofriendly solvent) as solvent.

Crystalline inclusion complex of Mirabegron with β-cyclodextrin Form II may also be defined by its preparation process. Accordingly, it is considered part of the invention the crystalline inclusion complex of Mirabegron and β-cyclodextrin Form II obtainable by any of the processes and specific conditions disclosed above.

Due to the high water solubility of the crystalline inclusion complexes of Mirabegron with β-cyclodextrin of the present invention, the characteristics of formulations containing them include a faster dissolution rate and shorter drug release time, as well as more efficient absorption. This translates into greater oral bioavailability of Mirabegron and an increase in biological activity, which may result in a reduction in drug dosage. Accordingly, it is also part of the invention a pharmaceutical composition comprising a therapeutically effective amount of a crystalline inclusion complex of Mirabegron with β-cyclodextrin as defined above, in particular Form I, Form II, or a mixture thereof, together with appropriate amounts of pharmaceutical excipients or carriers. The pharmaceutical composition may be used for oral administration or parenteral administration. Examples of the pharmaceutical composition for oral administration include tablets, pills, capsules, granules or powders. Appropriate excipients for the pharmaceutical composition include diluents, disintegrants, lubricants or and/or binders. If desired, the tablets may be coated with sugar coat or with gastric or enteric coating. An example of the pharmaceutical composition for parenteral administration is an inhaling composition.

It is also part of the invention the crystalline inclusion complexes of Mirabegron with β-cyclodextrin as defined above, in particular, Form I, Form II, or a mixture thereof for use as a medicament.

More in particular, it is part of the invention the crystalline inclusion complexes of Mirabegron with β-cyclodextrin of the present invention for use in the prevention and/or treatment of overactive bladder. This aspect may also be formulated as the use of any of the crystalline inclusion complexes of Mirabegron with β-cyclodextrin as defined above, in particular, Form I, Form II, or a mixture thereof, for the manufacture of a medicament for the treatment and/or prevention of overactive bladder. The present invention also relates to a method for the treatment and/or prevention of overactive bladder, comprising administering a pharmaceutically effective amount of any of the crystalline inclusion complexes of Mirabegron with β-cyclodextrin as defined above, in particular Form I, Form II, or a mixture thereof, together with pharmaceutically acceptable excipients or carriers, in a subject in need thereof, including a human.

Finally, as mentioned above, the new stable solid forms with increased solubility may also be useful for the preparation of other crystalline forms. Accordingly, it is also part of the invention the use of any of the crystalline inclusion complexes of Mirabegron with β-cyclodextrin defined above, in particular, Form I, Form II, or a mixture thereof as intermediates for the preparation of another Mirabegron crystalline Form, in particular Mirabegron Form α.

All the embodiments mentioned above or below are also disclosed optionally in combination with one or more features or the other embodiments defined below or above.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### BRIEF DESCRIPTION OF THE DRAWINGS

FIG. 1 shows the XRPD of the crystalline inclusion complex of Mirabegron with β-cyclodextrin Form I.
FIG. 2 shows the XRPD of the crystalline inclusion complex of Mirabegron with β-cyclodextrin Form II.

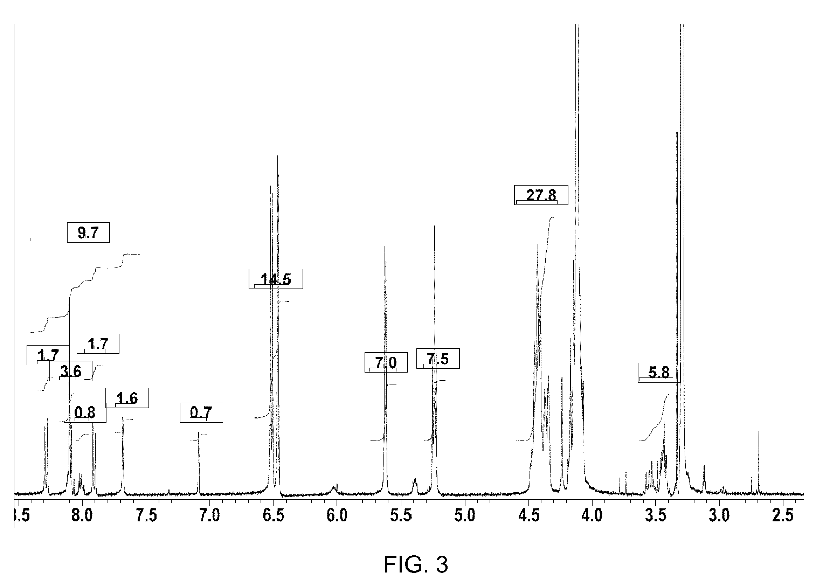

FIG. 3 shows the ¹H NMR analysis of the crystalline inclusion complex of Mirabegron with β-cyclodextrin Form I.
FIG. 4 shows the ¹H NMR analysis of the crystalline inclusion complex of Mirabegron with β-cyclodextrin Form II.

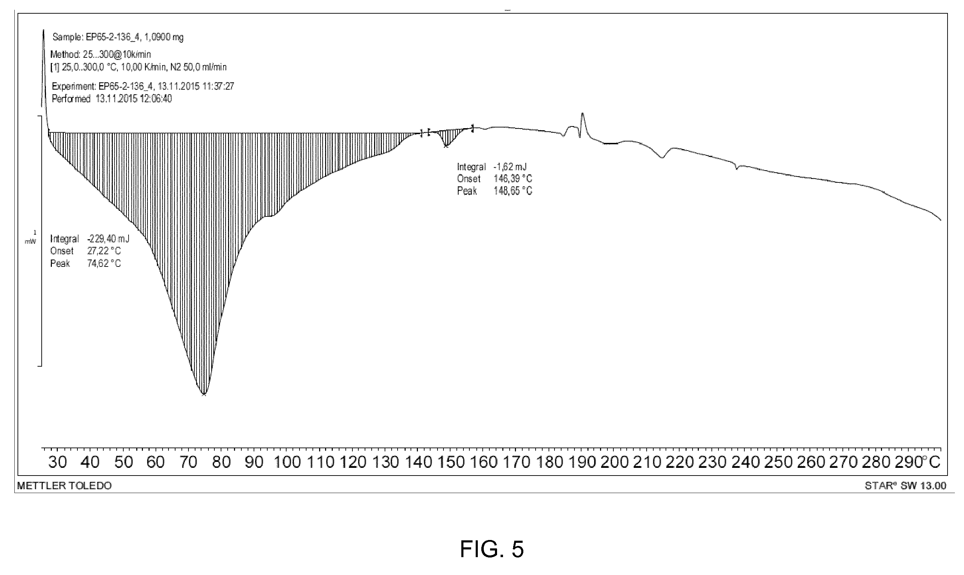

FIG. 5 shows the DSC analysis of the crystalline inclusion complex of Mirabegron with β-cyclodextrin Form I.
FIG. 6 shows the DSC analysis of the crystalline inclusion complex of Mirabegron with β-cyclodextrin Form II.

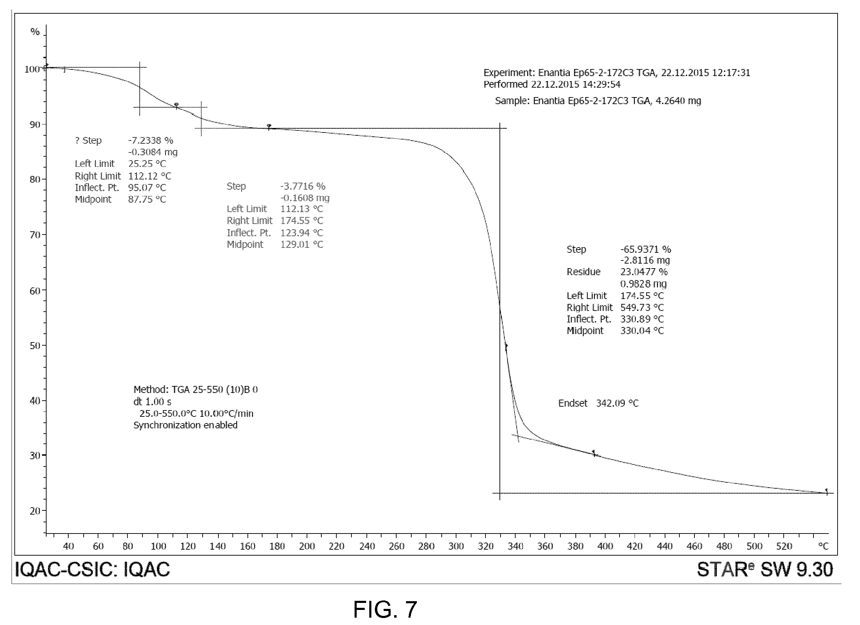

FIG. 7 shows the TGA analysis of the crystalline inclusion complex of Mirabegron with β-cyclodextrin Form I.

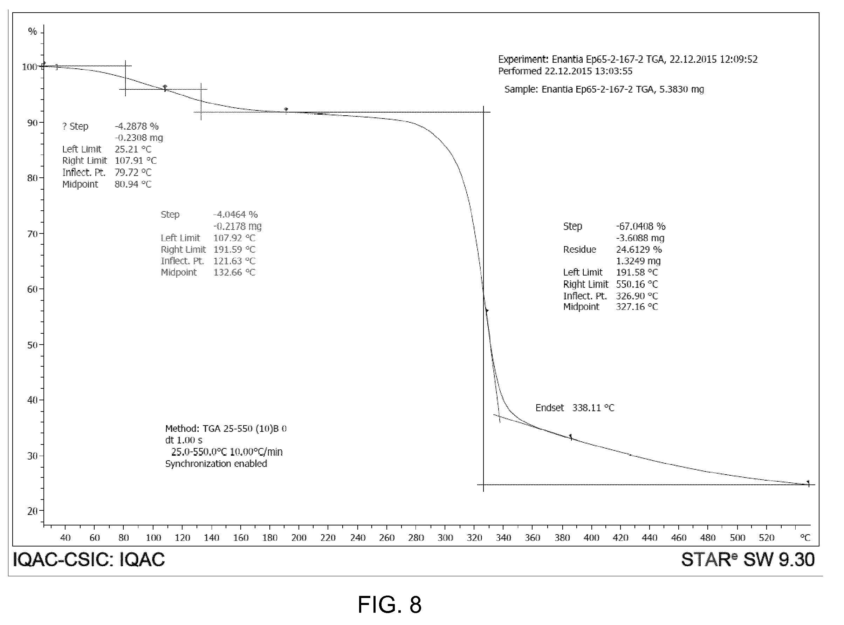

FIG. 8 shows the TGA analysis of the crystalline inclusion complex of Mirabegron with β-cyclodextrin Form II.

### EXAMPLES

XRPD analyses were performed using a PANalytical X'Pert diffractometer with Cu Kα radiation in Bragg-Brentano geometry. The system is equipped with a monodimensional, real time multiple strip detector. The diffractogram was recorded from 3° to 40° (2θ) at a scan rate of 0.0205° per second.

Proton nuclear magnetic resonance analyses were recorded in deuterated dimethyl sulfoxide (DMSO) in a Varian Mercury 400 spectrometer, equipped with a broadband probe ATB 1 H/19F/X of 5 mm. Spectra were acquired dissolving 5-10 mg of sample in 0.6 mL of deuterated solvent.

DSC analyses were recorded with a Mettler Toledo DSC2. Samples were weighed into 40 µL aluminum crucible with a pinhole lid and were heated, under nitrogen (50 mL/min), at 10°C/min from 25-30 to 300 °C.

Karl Fisher analyses were recorded with a Metrohm 787 KF Trinito apparatus using Hydranal-Composite 5 (Fluka Ref. 34805-500mL-R) as a reactant and Hydranal Methanol Rapid (Fluka Ref. 37817-1 L-R) as solvent in the titration cell. A minimun of 2 duplicates were analyzed for each sample.

Thermogravimetric analysis (TGA) was recorded in a thermogravimetric analyzer Mettler TGA/SDTA851e. Each sample was weighed into a 100 µL aluminium crucible and sealed with a lid. Samples were heated at 10 °C/min from 25 to 550 °C, under nitrogen (50 mL/min).

### Example 1: Preparation of crystalline inclusion complex of Mirabegron with β-cyclodextrin Form I

In an assay tube, 20 mg of Mirabegron (0.05 mmol) and 75 mg of beta-cyclodextrin (0.07 mmol, 1.3 eq) were suspended in water (0.3 mL) before heating to reflux. The resulting clear yellowish solution was cooled slowly to room temperature and a solid precipitated. Then the suspension was stirred for 6 h before filtering through a sintered glass funnel and washing with water (1x0.1 ml). The resulting solid was vacuum dried (5-10 mbar) at RT for 3h (time needed to dry Form I, the time is controlled by K-F) yielding 53 mg of an off white solid corresponding to Form I (Yield = 62 %). KF analisis of this solid indicates 10.8 % water content.

The inclusion complex of Mirabegron with β-cyclodextrin Form I shows XRPD as in FIG. 1; ¹H NMR, as in FIG. 3; DSC as in FIG. 5.

### Example 2: Preparation of crystalline inclusion complex of Mirabegron with β-cyclodextrin Form II

In an assay tube, 30 mg of Mirabegron (0.076 mmol) and 170 mg of beta-cyclodextrin (0.15 mmol, 2 eq) were suspended in water (0.5 mL) before heating to reflux. The resulting clear yellowish solution was cooled to RT and a solid precipitated. Then the suspension was stirred for 20 h before filtering through a sintered glass funnel and washing with water (0.1 ml). The resulting solid was vacuum dried (5-10 mbar) at room temperature for 20h (time needed to observe the complete transformation of Form I to Form II, the conversion is controlled by XRPD analysis and/or K-F) yielding 124 mg of a yellowish solid corresponding to Form II (Yield = 97 %). KF analisis of this solid indicates 8.9 % water content (aproximaely 8 water molecules).

The crystalline inclusion complex of Mirabegron with β-cyclodextrin Form II shows an XRPD as in FIG. 2, ¹H NMR as in FIG. 4, and DSC as in FIG. 6.

### Example 3: Preparation of crystalline inclusion complex of Mirabegron with β-cyclodextrin Form II

In an assay tube, 20.3 mg of Mirabegron (0.05 mmol) and 57.1 mg of beta-cyclodextrin (0.05 mmol, 1 eq) were suspended in water (0.4 mL). The suspension was stirred for 20 h before filtering through a sintered glass funnel and washing with water (0.1 ml). The resulting solid was dried dried under vacuum (5-10 mbar) at room temperature for 8 h (time needed to observe the complete transformation of Form I to Form II, the conversion is controlled by XRPD and/or K-F) yielding 56 mg of a yellowish solid corresponding by XRPD to crystalline inclusion complex of Mirabegron with β-cyclodextrin Form II with Mirabegron alpha (minoritary) (Yield= 66 %).

### Example 4: Preparation of crystalline inclusion complex of Mirabegron with β-cyclodextrin Form II by drying

A drying of crystalline inclusion complex of Mirabegron with β-cyclodextrin Form I was carried out under vacuum at 60 °C in the presence of P₂O₅, under these conditions after 15h partial transformation into Form II was observed by XRPD. The drying conditions were maintained under complete transformation.

### Example 5: Preparation of a mixture of crystalline inclusion complex of Mirabegron with β-cyclodextrin Form I and Form II by grinding

In an Eppendorf microtube, were added 20.7 mg of Mirabegron (0.05 mmol), 57.2 mg of β-cyclodextrin (0.05 mmol, 1 eq), two drops of water and 3 steel balls before grinding the resulting mixture in a ball mill (3 cycles of 15 min at 30Hz). The resulting solid was dried under vacuum at room temperature and analyzed by XRPD yielding to a mixture of Form I and Form II.

### Example 6: Obtention of crystalline inclusion complex of Mirabegron with β-cyclodextrin Form I from crystalline inclusion complex of Mirabegron with β-cyclodextrin Form II

Form II (c.a. 20 mg) was atmospherised in a chamber room at T = 40 °C and 75 ± 5 RH %, the sample was allowed to equilibrate under these conditions and transformation of Form II to I was controlled by XRPD. After 2 days, pure Form I, by XRPD analysis, was recovered.

### Example 7: Stability of crystalline inclusion complexes of Mirabegron with β-cyclodextrin Forms I and II

Stability studies of Forms I and II were performed with three conditions:
a) Atmospheric conditions (Room temperature under 40-60% RH)
b) Accelerated stability conditions described by ICH Guideline (40°C - 75% RH +/- 5%)
c) Dry stability conditions (40 °C - 15% RH +/- 5%)

The results for Form I are summarized in Table 3:

**Table 3. Stability study of crystalline inclusion complexes of Mirabegron with β-cyclodextrin Form I**

| **Time (days)** | **40-60% RH - RT** |
|---|---|
| **1** | Form I |
| **2** | Form I |
| **3** | Form I |
| **6** | - |
| **7** | - |
| **10** | Form I |
| **14** | Form I |
| **86** | Form I |
| **144** | Form I |
| **163** | Form I |

Under atmospheric conditions (23-26°C, 40-60% RH), Form I remained stable.

The results for crystalline inclusion complexes of Mirabegron with β-cyclodextrin Form II are summarized in Table 4:

**Table 4. Stability study of crystalline inclusion complexes of Mirabegron with β-cyclodextrin Form II**

| **Time (days)** | **40-60% RH - RT** |
|---|---|
| **1** | Form II |
| **2** | Form II |
| **3** | Form II |
| **6** | - |
| **7** | - |
| **10** | Form II |
| **14** | Form II |
| **79** | Form II |
| **137** | Form II |
| **156** | Form II |

Under atmospheric conditions (23-26°C, 40-60% RH), Form II remained stable.

Form II was also submitted to the following drying conditions: 60 °C, vacuum (5-10 mbar), in the presence of P₂O₅. Under these conditions after 15h Form II remained stable.

### Example 8: Solubility study of crystalline inclusion complexes of Mirabegron with β-cyclodextrin

A water solubility study at room temperature was performed comparing Mirabegron Form α and crystalline inclusion complexes of Mirabegron with β-cyclodextrin Forms I and II by HPLC analysis of saturated water solutions.

Crystalline inclusion complexes of Mirabegron with β-cyclodextrin Form I_(20 mg) was stirred in 0.8 ml of water (according to stoichiometry c.a. 16.6 mg of β-cyclodextrin per ml) for 19h. A slurring of Mirabegron Form α was performed under the same conditions. In both cases, a sample was centrifuged and after decantation the mother liquor was analyzed by HPLC indicating a peak area corresponding to Mirabegron of: 47430 counts in the case of Form I and 1728 counts in the case of Mirabegron Form α.

Therefore, there was a 27 fold increase of solubility of the crystalline inclusion complex of Mirabegron with β-cyclodextrin Form I with respect to Form α of Mirabegron under these conditions.

Crystalline inclusion complexes of Mirabegron with β-cyclodextrin Form II (24 mg) was stirred in 1 ml of water (according to stoichiometry c.a. 16 mg of cyclodextrin per ml) for 19h. A slurring of Mirabegron Form α (20 mg) was performed in the same conditions. In both cases, a sample was centrifuged and the mother liquor was analyzed by HPLC indicating a peak area corresponding to Mirabegron of: 21471 counts in the case of Form II and 1570 counts in the case of Mirabegron Form α. Therefore, there was a 13.5 fold increase of solubility under these conditions.

Therefore, according to HPLC area, an important increase of Mirabegron solubility in comparison to solubility of Mirabegron Form α was observed for both forms: Form II: 14 times more soluble than α-Form and Form I: 27 times more soluble than α-Form.

The solid recovered at the end of the solubility study of Form II was Form I. The difference of solubility observed for both forms should be due to kinetic factor (thermodynamic equilibrium was not reached). This transformation of Form II into Form I in water confirms that form II is probably a less hydrated form with the same stoichiometry than Form I.

Form I is stable in suspension in pure water at room temperature.

### REFERENCES CITED IN THE APPLICATION

- EP1028111A1
- EP1440969A1
- WO2012156998A2
- Betmiga Mirabegron report (AusPar), Myrbetriq Highlights of prescribing information (FDA)
- Characterization of stress degradation products of mirabegron using UPLC-QTOF-MS/MS and in silico toxicity predictions of its degradation products", P. D. Kalariya et al.; RSC Adv., 2015,5, 31024-31038
- Pharmaceutical Applications of Cyclodextrins. 1. Drug Solubilization and Stabilization", T. Loftsson et al. J. Pharm. Sc. 1996, vol. 85, pp.1017- 1025

## Claims

1. A crystalline inclusion complex of Mirabegron with β-cyclodextrin.

2. The crystalline inclusion complex according to claim 1, which is a hydrated form.

3. The crystalline inclusion complex according to any of the claims 1-2, wherein the Mirabegron : β-cyclodextrin molar ratio determined by ¹H NMR is 1:1.

4. The crystalline inclusion complex according to any of the claims 1-3, which is named crystalline Form I and is **characterized by** having an X-ray diffractogram that comprises characteristic peaks at approximately 7.2, 9.9, 12.0, 14.5, 15.7, 17.6, 18.5, 18.8, 20.7, and 23.8 degrees 2 theta at a Cu-K_{α} radiation, A = 1.5406 Å.

5. The crystalline inclusion complex Form I according to claim 4, which is **characterized by** having an X-ray diffractogram that further comprises characteristic peaks at approximately 6.0, 7.2, 9.9, 12.0, 14.5, 15.7, 17.6, 17.7, 18.5, 18.8, 19.7, 20.7, 21.4, 22.6, 23.3, 23.8, 25.9, 29.4, 36.1, and 38.1 degrees 2 theta (Cu-K_{α} radiation, λ = 1.5406 Å).

6. The crystalline inclusion complex according to any of the claims 1-3, which is named crystalline Form II and is **characterized by** having an X-ray diffractogram that comprises characteristic peaks at approximately 5.8, 6.6, 6.9, 11.0, 11.6, 11.9, 14.2, 15.5, 16.7, 17.0, 17.4, 18.0, 18.4, 19.2, and 20.8 degrees 2 theta at a Cu-K_{α} radiation, A = 1.5406 Å.

7. The crystalline inclusion complex Form II according to claim 6, which is **characterized by** having an X-ray diffractogram that further comprises characteristic peaks at approximately 5.8, 6.6, 6.9, 11.0, 11.6, 11.9, 13.3, 14.2, 15.0, 15.3, 15.5, 16.7, 17.0, 17.4, 18.0, 18.4, 19.2, 20.8, and 23.5 degrees 2 theta (Cu-K_{α} radiation, λ = 1.5406 Å).

8. The crystalline inclusion complex according to claim 1, which is a mixture of crystalline Form I and crystalline Form II.

9. A crystalline inclusion complex of Mirabegron with β-cyclodextrin as defined in any of the claims 1-8, for use as a medicament.

10. A crystalline inclusion complex of Mirabegron with β-cyclodextrin as defined in any of the claims 1-8, for use in the prevention and/or treatment of overactive bladder.

11. A pharmaceutical composition comprising a therapeutically effective amount of a crystalline inclusion complex of Mirabegron with β-cyclodextrin, as defined in any of the claims 1-8, together with appropriate amounts of pharmaceutical excipients or carriers.

12. A process for the preparation of a crystalline inclusion complex of Mirabegron with β-cyclodextrin Form I as defined in any of the claims 4-5, which comprises:
a1) Crystallizing an inclusion complex of Mirabegron with β-cyclodextrin either from a solution comprising Mirabegron and β-cyclodextrin in water, or from a solution comprising Mirabegron and β-cyclodextrin in a solvent system which is a mixture of a water miscible solvent/water; or alternatively,
a2) Slurring Mirabegron and β-cyclodextrin in water at a temperature of from room temperature and 40 °C for the necessary period of time until complete formation of an inclusion complex:
b) Filtrating the crystalline inclusion complex Form I thus obtained and drying at room temperature under vacuum the product thus obtained for the necessary period of time to obtain dry Form I.

13. A process for the preparation of a crystalline inclusion complex of Mirabegron with β-cyclodextrin Form II as defined in any of the claims 6-7, which comprises
a1) Crystallizing an inclusion complex of Mirabegron with β-cyclodextrin either from a solution comprising Mirabegron and β-cyclodextrin in water, or from a solution comprising Mirabegron and β-cyclodextrin in a solvent system which is a mixture of a water miscible solvent/water; or alternatively,
a2) Slurring Mirabegron and β-cyclodextrin in water at a temperature of from room temperature to 40 °C for the necessary period of time until complete formation of an inclusion complex: and
b) Filtrating the crystalline inclusion complex Form I thus obtained and drying at a temperature of from room temperature to 80° C under vacuum the product thus obtained until complete transformation to Form II.

14. The process according to claim 13, wherein the drying temperature is of from 40-80 °C.

15. A process for preparing the crystalline inclusion complex of Mirabegron with β-cyclodextrin Form II as defined in any of the claims 6-7, which comprises submitting the crystalline inclusion complex of Mirabegron and β-cyclodextrin Form I defined in any of the claim 4-5 to a drying step under vacuum at a temperature of from room temperature to 80 °C, optionally, in the presence of a desiccant agent.
